# EUROPEAN PATENT APPLICATION

(11) **EP 1 674 573 A1**
(43) Date of publication of application: **28.06.2006**
(21) Application number: 04405800.6
(22) Date of filing: 23.12.2004
(51) Int. Cl.: C12N 15/11, C07H 19/04, C07H 19/16, C07H 21/02

(54) **RNA-sequence or nucleoside/nucleotide analogues and method for producing the analogues**

(71) Applicant: ECOLE POLYTECHNIQUE FEDERALE DE LAUSANNE, 1015 Lausanne (CH)
(72) Inventor: Pitsch, Stefan, 1004 Lausanne (CH); Muthuppalaniappan, Meyyappan MP Meyyappan, Cheppakkam Chennai 600-005 Tamilnadu (IN); Porcher, Sébastien, 1007 Lausanne (CH)
(74) Representative: Frei Patent Attorneys

(57) **Abstract**

Nucleic acid sequence analogues, in particular tRNA analogues comprising in the place of a 3'-terminal adenosine a 3'-terminal 2'-deoxy-2'-thioadenosine are able to react site-specifically with weakly activated amino acid derivatives (e.g. phenylthioesters of amino acids), thereby connecting the amino acid to the 2'-thiol-group of the 3'-terminal thioadenosine via a thioester bond. A fast and thermodynamically favored intramolecular transesterification reaction leads then to the formation of the corresponding 3'-O-acyl-derivative. A correspondingly modified tRNA is an active analogue of an aminoacylated tRNA and allows introduction of the amino acid bound thereto into proteins. The aminoacylated tRNA analogue according to the invention can be isolated in pure form under acidic conditions (pH 5.0). At pH 7.4 and 25°C it is found to have a half-life of 25 min, which compares well with the half-life of 40 min for the corresponding native compound.

## Description

### Field of the invention

The invention belongs to the field of molecular biology and refers to RNA-sequences or nucleoside/nucleotide analogues, in particular to tRNA analogues applicable for ribosome-mediated incorporation of unnatural amino acids in proteins. The invention further refers to methods for producing the analogues.

### Background of the invention

Ribosomal biosynthesis of proteins according to RNA-sequence information, which is again obtained from DNA-sequence information (genes), is one of the central events in each cell of each living being. In this process, named translation, genotypic information (nucleobase-sequence information stored in DNA and RNA, respectively) is translated into phenotypic information (amino acid sequences), resulting in proteins with different structures and functions.

Translation is a very complex process which includes the concerted action of more than 100 macromolecules. Key-compounds and key-complexes in this process are: the messenger RNAs (mRNAs), the transfer RNAs (tRNAs), the ribosomes and the elongation factors.

Messenger RNAs are RNA-copies (transcripts) of specific DNA-sequences (genes). After their synthesis, but before they are used in translation, they often are extensively modified. The mRNAs carry the specific information required for the sequence-specific assembly of amino acids to specific proteins. The mRNAs consist of a linear sequence of four different building blocks (nucleotides), which can be assembled in any possible order. The proteins, in contrast, consist of linear sequences of 20 different building blocks (amino acids); each combination of these 20 amino acids leads to a different molecule with different structure and different function. In order to encode 20 different amino acids with only four different nucleotides, sequences of three nucleotides are used as signal; they are named codons and there are 4³ = 64 different combinations of them. One of these codons is used as a start-signal (during initiation of translation), three of them are used as stop-signals (during termination of translation) and the remaining 60 codons are all encoding for amino acids. There is at least one specific codon for each of the 20 amino acids; some of them are encoded by several codons, but no codon encodes for more than one amino acid.

Transfer RNAs are relatively short RNA-sequences which consist of 56 to 95 nucleotides (typical length 76 nucleotides). In addition to the four canonical RNA-nucleotides, they contain a large variety of sugar- and nucleobase-modified nucleotides, some of them highly conserved among all known tRNAs. Despite their different lengths, sequences and modifications, they have geometrically very similar three-dimensional, L-shaped, structures; the distance between the ends of the two arms of these "Ls" is always about 100 Angstroms. The end of the long arm contains a three-nucleotide sequence, named the anticodon, which is complementary to the three-nucleotide codon-sequences of the mRNAs. At the end of the short arm, a specific amino acid is attached enzymatically. Consequently, the tRNAs are at the same time carrying amino acids and decoding the codon-information of the mRNAs, thereby providing the link between information (mRNA-sequence) and sequence-specific assembly of proteins from amino acids. The highly accurate loading of a given tRNA with the corresponding amino acid is carried out by enzymes, named aminoacyl-tRNA-synthetases, which first activate a specific amino acid by the help of ATP, secondly bind a specific tRNA and thirdly connect the activated amino acid to the 3'-terminal (conserved) adenosine of the tRNA via an ester-bond. The accuracy of this step is highly controlled, because connection of an amino acid to a wrong tRNA would result in incorporation of a wrong amino acid into a protein.

Ribosomes are huge RNA/protein complexes consisting of two subunits, the large and the small subunit, respectively. Depending on the organism, both subunits consist of some RNA-sequences of different length (ribosomal RNA, rRNA) and many, rather small proteins. The ribosomes are responsible for bringing together the mRNAs and the aminoacylated tRNAs and for catalyzing the formation of proteins from the amino acids attached to the tRNAs. With the help of several proteins (initiation factors) and a specific aminoacylated tRNA (with an anticodon complementary to the start-codon of an mRNA and a methionine or N-formylmethionine as amino acid) the two subunits are assembled on the 5'-end of the mRNA and translation can start. The elongation step is carried out by first binding a aminoacylated tRNA with an anticodon complementary to the codon following the start-codon. Next, the amino-group of the amino acid of the aminoacylated tRNA is attacking the carbonyl-group of the methionine (or *N*-formylmethionine) bound to the first tRNA, thereby forming the first peptide-bond of the protein to be synthesized and releasing the methionine from its tRNA. This free (non-aminoacylated) tRNA is now released, the other tRNA, carrying now a dipeptide is translocated (together with the mRNA) and another elongation step takes place in a completely analogous manner. Thereby the peptide is formed by stepwise assembly of each amino acid. This process is going on until one of the three stop-codons is reached. Since no complementary tRNA is present, the translation stops and eventually a release-factor (a protein with the shape of a tRNA) binds to the ribosome and the stop-codon of the mRNA, and cleaves the ester bond between the full-length protein and the last tRNA by hydrolysis. The resulting complex dissociates into the two ribosomal subunits, the mRNA and the protein.

Elongation factors are proteins which strongly bind both the amino acid and part of the RNA of aminoacylated tRNAs, thereby stabilizing the fragile, energy-rich ester bond (t_{1/2} of hydrolysis under physiological conditions: 5-20 min) formed between the tRNAs and the amino acids. Once arrived at the ribosome and before the aminoacylated tRNA is entering the ribosome, the complex formed between the aminoacylated tRNA and the elongation factor is released with concomitant hydrolysis of a GTP bound to the elongation factor.

There is a considerable interest in tRNAs which are loaded with unnatural amino acids. On several occasions, it has been shown that such amino acids can be incorporated site-specifically into proteins, e.g. leading to cross-linking reactions with components of membranes or to be used for fluorescence measurements [2], leading to enzymes with different catalytic or structural properties than the wild-type enzyme [3] or to caged proteins which regain their function after photolytic deprotection [4]. In general, this methodology allows the site-specific introduction of almost every feasible amino acid into proteins and is an extremely important tool for the study of protein-interactions, for functional and structural assays and for the development of designer-enzymes with artificial properties [5].

The ribosome-mediated incorporation of unnatural amino acids into proteins is e.g. carried out using a combination of genetically engineered DNA (which is transcribed enzymatically into the corresponding mRNA) and an engineered suppressor-tRNA [6]. The latter carries the desired amino acid and has an anticodon-site which is complementary to a natural stop codon introduced site-specifically into the DNA. In this way, the codon usually leading to a stop of the translation process serves as an active codon, allowing incorporation of the unnatural amino acid into the growing peptide chain. Artificially aminoacylated tRNAs are obtained by enzymatic ligation of a truncated tRNA (produced by T7-polymerase mediated transcription of an appropriate DNA-construct) with an aminoacylated dCA-dimer [7][8]. This semisynthetic method is usually carried out on small scale. It gives a tRNA-analogue, containing only the four canonical nucleosides; this stands in contrast to natural tRNAs which contain a multitude of modified nucleosides [9].

A different approach is designed as *in-vivo* process and involves the development of artificial aminoacyl-tRNA-synthetases which recognize the artificial tRNA and connect it to an artificial amino acid [10]. This method is in principle very elegant but restricts the choice of the amino acid and has to be adapted to every new combination of tRNA and amino acid. The latest approaches involve ribozymes which catalyze the transfer of the amino acid of short aminoacylated RNA-fragments to a tRNA [11], or short peptide nucleic acid (PNA) sequences, which are complementary to the 3'-end of the tRNA, and carry an activated amino acid (connected via a thioester bond and a linker with the PNA), which, after duplex formation, is then transferred to the tRNA [20].

During the last years, two concepts to overcome the limitations of the initial suppressor-tRNA approach (based entirely on stop-codons) were developed. The first includes the simultaneous incorporation of unnatural (orthogonal) complementary nucleotides, such as isoC/isoG [12] and a thienyl-substituted 2-aminopurine/pyridine-2-one [13] into the tRNA and the mRNA respectively. Thereby new (and orthogonal) codons were created and successfully exploited in the above-mentioned context. The second concept is based on tRNA/mRNA pairs containing four or five complementary RNA-nucleotides as anticodon- and codon-analogues, respectively [14][15].

The first concept is experimentally more difficult to realize than the second, and consequently only two examples are known so far. In both approaches, the modified mRNA was obtained by transcription of a modified DNA (the modifications were introduced by ligation with a chemically synthesized DNA-fragment), whereas the corresponding tRNA was prepared by ligation of a chemically synthesized RNA-fragment with an enzymatically produced fragment. This approach is very labor-intensive (multi-step preparation of the aminoacylated tRNA) and restricted to modifications, which are transcribed efficiently. The second concept, involving four- or five-nucleotide-codons/anticodons is in principle very simple to realize by standard molecular-biology methods and was already used for the simultaneous introduction of two unnatural amino acids into the same protein [15]. However, a certain limitation is the requirement to eliminate the corresponding triplets from the mRNA.

As taken from the above paragraphs, there are currently five methods for the preparation of aminoacylated tRNAs containing unnatural amino acids. These are in short:
1. Regioselective esterification of the diol-moiety of a p-CA-dimer with N-protected, activated amino acid derivatives, followed by ligation to a truncated tRNA and removal of the amino acid protecting group (advantage: general method, several examples known; disadvantage: rather inefficient, multi-step synthesis, unstable intermediates);
2. Ribozyme-mediated transfer of the amino acid from a short aminoacylated RNA-fragment to a tRNA (advantage: probably quite general method; disadvantage: complicated system, presumably complex kinetics, isolation of product difficult, difficult preparation of aminoacylated RNA-fragments, presumably not always selective);
3. Transfer of an amino acid attached via a thioester bond and a linker to short peptide nucleic acid (PNA) sequences, which are complementary to the 3'-end of the tRNA (advantage: probably quite general method; disadvantage: complicated system, presumably complex kinetics, difficult preparation of PNA amino acid conjugates);
4. Creating derivatives of aminoacyl-tRNA-synthetases, which selectively recognize an unnatural tRNA and an unnatural amino acid and catalyze the formation of the corresponding unnatural (orthogonal) aminoacylated tRNA (advantage: highly efficient, applicable *in vivo* and *in vitro;* disadvantage: very complicated and time-consuming process, not general, as for every amino acid a new synthetase analogue has to be created);
5. Substitution of a natural amino acid by a related derivative which is recognized by a natural aminoacyl-tRNA-synthetase (advantage: very simple method, quite efficient; disadvantage: no site-selective incorporation possible, restricted to a few analogues of amino acids).

### Brief description of the invention

The object of the invention is to create a nucleic acid sequence or nucleoside/nucleotide analogue, in particular a tRNA analogue which is able to be aminoacylated in aqueous solution with a freely selected amino acid, wherein the aminoacylation reaction can be carried out without a specific catalyst such as a specific enzyme or ribozyme and without the requirement of preparing a short aminoacylated nucleic acid or peptide nucleic acid fragment and transferring the amino acid from the latter. The aminoacylated products according to the invention are to have a stability under physiological conditions which compares with the corresponding stability of natural 3'-*O*-aminoacylated tRNAs. The analogue according to the invention is in particular a tRNA analogue able to be aminoacylated in the 3'-position *in vitro* to form an aminoacyl-product applicable for *in vivo* (cultured cells or micro-organisms) or *in vitro* translation, i.e. for ribosome-mediated, site-specific incorporation of unnatural amino acids into proteins. Further objects of the invention are methods for producing the analogues.

The invention bases on the finding that nucleic acid sequence or tRNA analogues comprising in the place of a 3'-terminal adenosine a 3'-terminal 2'-deoxy-2'-thioadenosine are able to react site-specifically with weakly activated amino acid derivatives (e.g. phenylthio-esters of amino acids), thereby connecting the amino acid to the 2'-thiol-group of the 3'-terminal 2'-deoxy-2'-thioadenosine substituted RNA-sequence via a thioester bond. A fast and thermodynamically favored intramolecular transesterification reaction leads then to the formation of the corresponding 3'-O-acyl-derivative. A correspondingly modified tRNA is an active analogue of an aminoacylated tRNA and allows introduction of the amino acid bound thereto into proteins. The aminoacylated tRNA analogue according to the invention can be prepared under a variety of different conditions (pH values 7.4 - 4.5, temperatures 20° - 40°) and can be isolated in pure form under acidic conditions. At pH 7.4 and 25°C it is found to have a half-life of 25 min, which compares with the half-life of 40 min for the corresponding native compound [19].

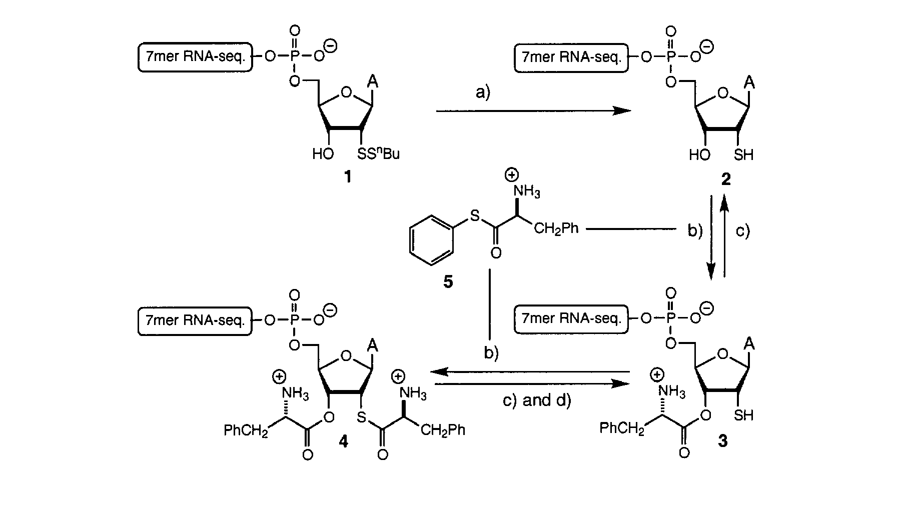

The above formula shows the aminoacylation reaction which is the basis of the invention. Therein a) is the release of the active species **2** (thiol-containing RNA-sequence) by reductive cleavage of the protected precursor **1** (disulfide) with a reducing agent (e.g. tris(carboxyethyl)phosphine, TCEP), preferentially under the same conditions (e.g. buffered aqueous solutions) under which the subsequent aminoacylation reaction is carried out (see Table 1 for specific conditions), and b) is the addition of an activated amino acid **5** (e.g. phenyl thioesters) to the thiol-modified nucleic acid **2** prepared in step a) and dissolved in aqueous buffers (see Table 1 for specific conditions and results). After formation of the monoacylated compound **3,** a subsequent acylation reaction can occur, which results in the formation of the diacylated derivative **4.** The ratio of compounds **3** and **4** depends on the reaction conditions and the reaction time (see Table 1). Steps c) are the hydrolysis reactions which strongly depends on conditions. At pH 7.4, hydrolysis of **3** to **2** occurs with a rate constant of k = 0.02 min⁻¹ and k = 0.075 min⁻¹, at 25° and 37°, respectively, and hydrolysis of **4** to **3** occurs with a rate constant of k = 0.15 min⁻¹ at 37°. Step d) is the selective cleavage of the diacylated derivative **4** to the monoacylated RNA-sequence **3** which is effected by addition of weak nucleophiles (e.g. NaN₃).

**Table 1: Course of the aminoacylation reaction under different conditions**

| Conditions¹⁾ | | | t = 10 min²⁾ | | | t = 50 min²⁾ | | |
|---|---|---|---|---|---|---|---|---|
| pH | **5** | T | **2** | **3** | **4** | **2** | **3** | **4** |
| | eq. | [°C] | [%] | | | | [%] | |
| 7.4 | 12 | 25 | 1 | 18 | 73 | 5 | 37 | 46 |
| 6.5 | 12 | 25 | 3 | 19 | 71 | 3 | 24 | 64 |
| 5.5 | 12 | 25 | 7 | 19 | 70 | <1 | 10 | 86 |
| 5.0 | 12 | 25 | 10 | 18 | 70 | <1 | 10 | 87 |
| 4.7 | 12 | 25 | 12 | 18 | 68 | <1 | 10 | 87 |
| 3.7 | 12 | 25 | 43 | 20 | 36 | 18 | 26 | 55 |
| 7.4 | 6 | 25 | 2 | 24 | 64 | 6 | 78 | 10 |
| 7.4 | 12 | 37 | 1 | 17 | 72 | 5 | 41 | 42 |
| 5.0 | 6 | 25 | 27 | 29 | 43 | 1 | 33 | 65 |
| 5.0 | 3 | 25 | 54 | 20 | 23 | 15 | 35 | 47 |
| 5.0 | 12 | 37 | 2 | 27 | 67 | 1 | 32 | 64 |
| 5.0 | 6 | 37 | 8 | 34 | 53 | 1 | 35 | 60 |
| 5.0 | 3 | 37 | 30 | 29 | 34 | 4 | 41 | 50 |

| | | | | | | | | |
|---|---|---|---|---|---|---|---|---|
| ¹⁾ Incubation of 1 (c = 0.085 mM, 40µl) with TCEP (c = 1.0 mM at pH 7.4, 6.5; 2.5 mM at pH 5.5, 5.0, 4.7; 10 mM at pH 3.7) in aq. buffers (50 mM each; pH 7.4: Tris-HCl, pH 6.5: H₃PO₄-NaOH; pH 5.5, 5.0, 4.7: AcOH-NaOH; pH 3.7: HCOOH-NaOH) at 25° for 30 min, followed by addition of 5 (indicated equivalents relative to 2) in DMF (4 µl). | | | | | | | | |
| ²⁾ t = incubation time; quantification by RP-HPLC at 260 nm; values relative to sum of all detected peaks. | | | | | | | | |

The mechanism of the aminoacylation reaction as shortly described above seems to have a mechanism which is similar to the mechanism of the site-specific ligation of oligopeptides by the so called "native chemical ligation" [1]. Both reaction mechanisms and the analogue mechanism for a 3'-terminal 3'-deoxy-3'-thioadenosine substituted tRNA are shown below.

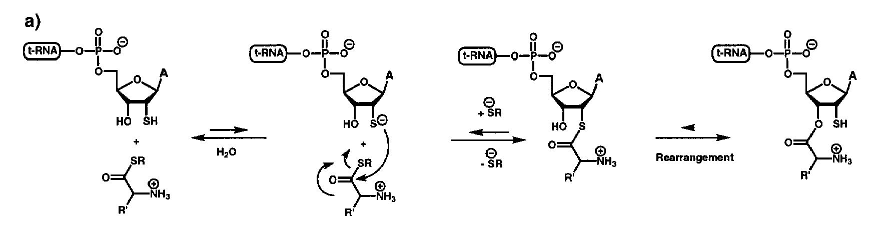

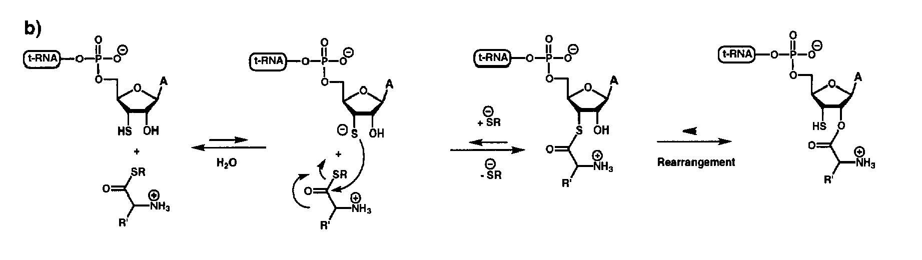

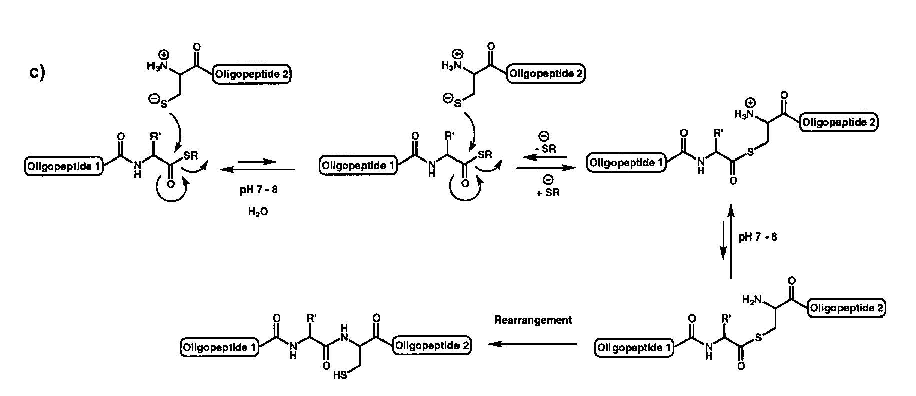

The above formulas show: a) the proposed mechanism for the aminoacylation of RNA-sequences containing a 3'-terminal 2'-deoxy-2'-thioadenosine with activated amino acids, b) the same reaction with RNA-sequences containing a 3'-terminal 3'-deoxy-3'-thioadenosine, and c) the mechanism of the "native chemical ligation" of oligopeptides.

As shown above, the aminoacylation process can be carried out not only with a 3'-terminal 2'-deoxy-2'-thioadenosine tRNA analogue but also with a 3'-terminal 3'-deoxy-3'-thioadenosine tRNA analogue and it can be carried out not only with a tRNA analogue but with any RNA-sequence analogue carrying a correspondingly modified 3'-terminal nucleoside or with a corresponding nucleoside/nucleotide. This nucleoside is not necessarily an adenosine but can also carry a substituted adenine or an other natural or substituted base. The selection of amino acids is not limited. Instead of the thiophenylester other activated amino acid derivatives can be used in the aminoacylation process, such as e.g. thioalkylester, nitrophenylester, pentafluorophenylester, N-hydroxy-succinimidester, or corresponding anhydrides (mixed or symmetrical).

For *in vivo* applications (cultures of cells or micro-organisms) of tRNA analogues according to the invention, it is suggested to carry out the aminoacylation process separately (in absence of the cells or micro-organisms, i.e. in vitro), to stabilize the aminoacylation product (e.g. under acidic conditions or by complexation with an elongation factor), to isolate the stabilized product and to finally introduce it into the cells or micro-organisms by conventional methods (transfection, microinjection).

For *in vitro* applications of tRNA analogues according to the invention, the aminoacylation reaction can either be carried out separately (as described above) and the resulting product can be introduced into the *in vitro* translation system, or the whole process (aminoacylation and translation) can be carried out in one pot by adding the activated amino acid derivative together with a catalytical amount of the tRNA analogue to the *in vitro* translation reaction.

### Brief description of the Figures

The invention is further described in connection with eight examples and the following Figs., wherein:

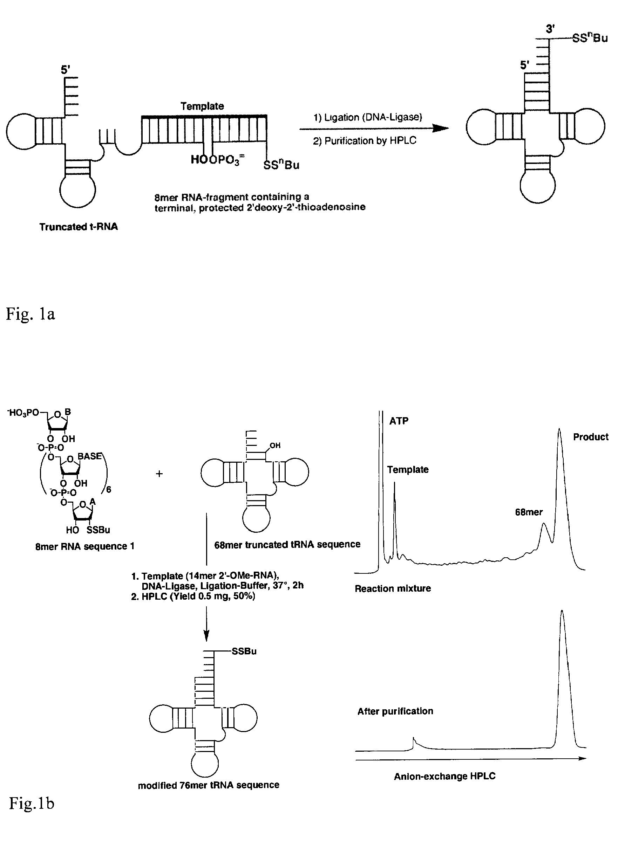

**Figs. 1a and 1b** show the preparation of a tRNA analogue containing a protected 3'-terminal 2'-deoxy-2'-thioadenosine nucleoside, the preparation comprising an enzymatic ligation of a 68mer RNA-transcript and an 8mer RNA-sequence analogue according to the invention with the aid of a 14mer 2'-OMe-RNA as a template;

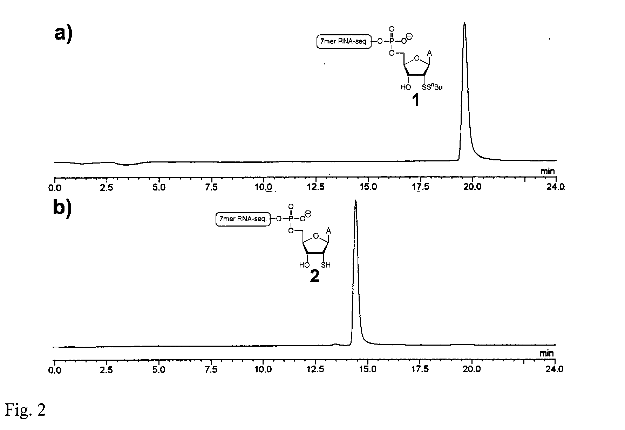

**Figs. 2a and 2b** illustrate the cleavage of the disulfide protection group from the 3'-terminal 2'-deoxy-2'-thioadenosine nucleoside of an RNA-sequence analogue **1** with TCEP at 25° and pH 7.4 to produce the deprotected analogue **2** with RP-HPLC traces of the of protected analogue **1** (Fig. 2a) and of the reaction mixture after 30 min reaction time (Fig. 2b);

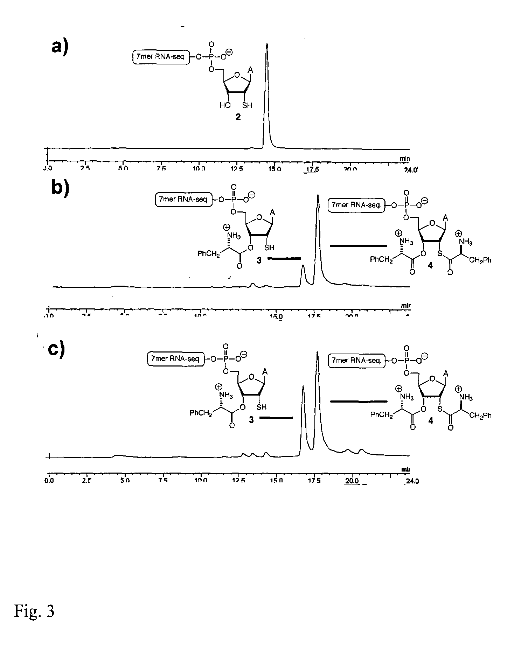

**Figs. 3a to 3c** illustrate the aminoacylation reaction of an 8mer RNA-sequence analogue **2** according to the invention with an activated amino acid, at pH 7.4 and 37° with RP-HPLC traces of the analogue **2** (Fig 3a) and of the reaction mixture after 2 min reaction time (Fig 3b) and after 20 min reaction time (Fig. 3c);

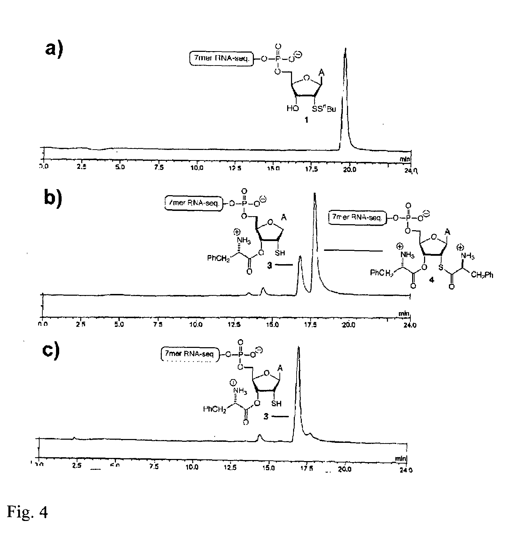

**Figs. 4a to 4c** illustrate with RP-HPLC traces the cleavage of the disulfide protection group from the 3'-terminal 2'-deoxy-2'-thioadenosine nucleoside of an 8mer RNA-sequence analogue **1** (Fig. 4a) with TCEP at 25° and pH 5.5 for 30 min, and the 3'-*O*-aminoacylation reaction with an activated amino acid at pH 7.4 and 37° for 30 min (Fig 4b), and the almost selective formation of the monoacylated product **3** upon treatment with NaN₃ at 25° for 30 min and desalting on a size exclusion cartridge (Fig 4c);

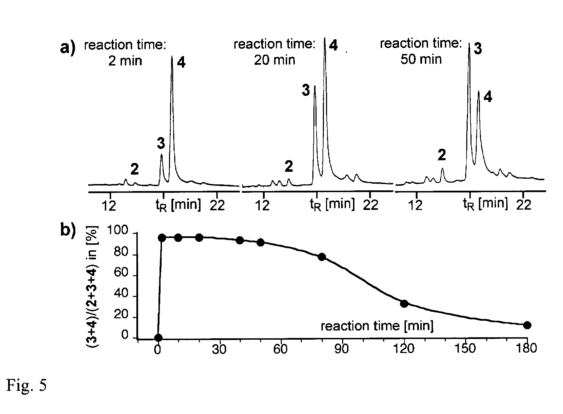

**Figs. 5a to 5b** illustrate the time course of an aminoacylation reaction of a 8mer RNA-sequence analogue according to the invention **(2** in Fig. 3) with an excess (12 equivalents) of an activated amino acid at pH 7.4 and 37° with RP-HPLC traces for reaction times of 2, 10 and 50 min, respectively (Fig. 5a) and the relation between the amount of the sum of aminoacylated products **(3+4** in Fig. 3) to the sum of all assigned RNA-sequences **(2+3+4** in Fig 3) versus the reaction time (Fig. 5b);

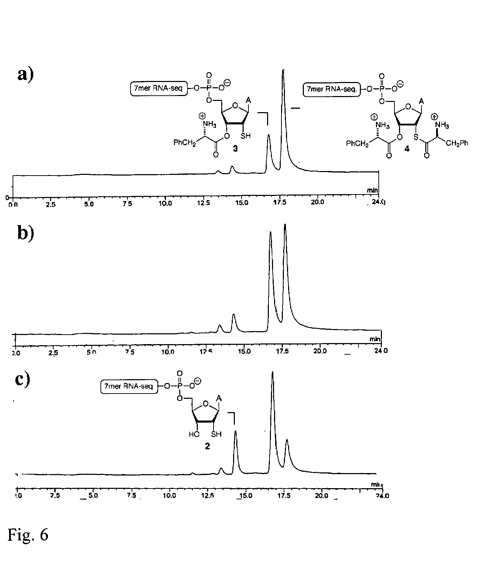

**Figs. 6a to 6c** show RP-HPLC traces of reaction mixtures obtained by incubating the desalted reaction mixture (trace of Fig 6a) in 0.1M aq. Tris-HCl (pH 7.4) at 37°, as analyzed after 2 min (Fig 6b) and 8 min (Fig 6c);

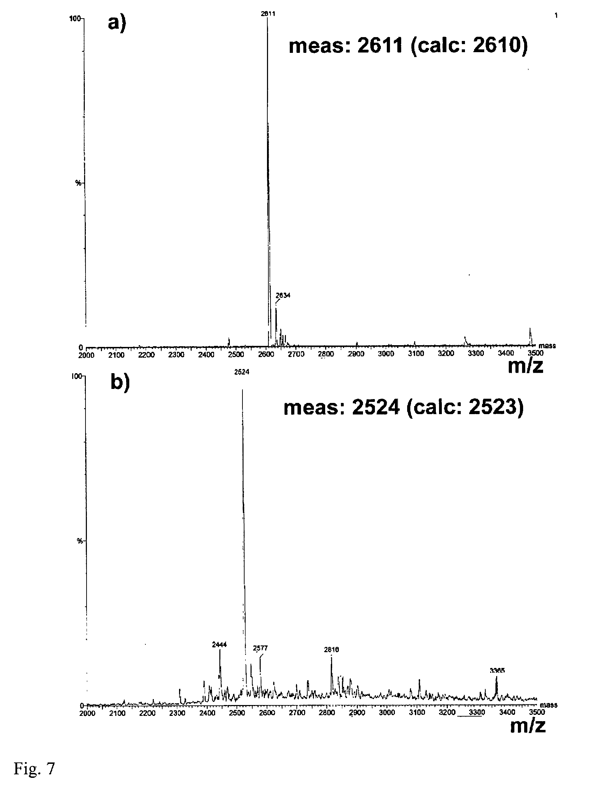

**Figs. 7a to 7b** show ESI-MS spectra (neg. mode) of the starting material, i.e. the protected RNA-sequence analogue **(1** in Fig 3) prepared according to the invention (Fig. 7a) and of the thiol substituted RNA-sequence analogue **(2** in Fig 3), obtained by treatment of **1** with TCEP (Fig. 7b);

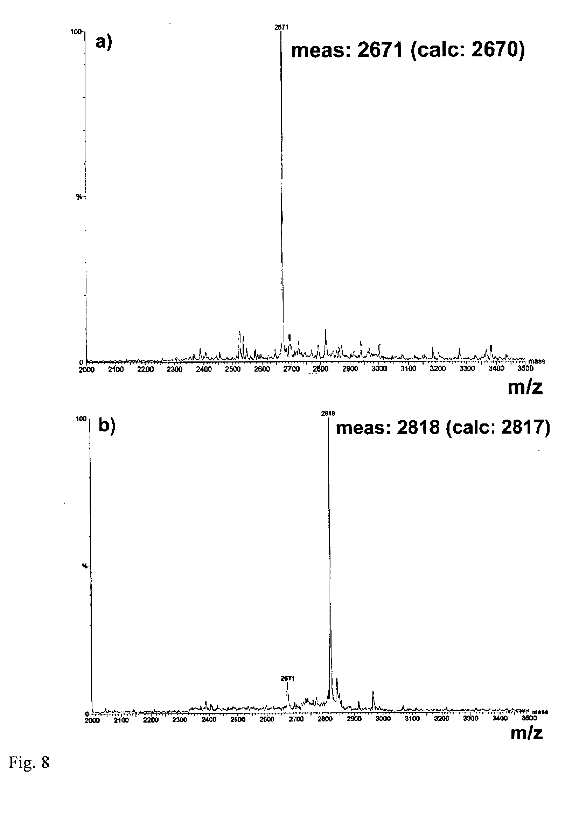

**Figs. 8a to 8b** show ESI-MS spectra (neg. mode) of the monoacylated RNA-sequence **(3** in Fig 3), isolated by RP HPLC (Fig. 8a), and of the diacylated RNA-sequence **(4** in Fig 3; containing ca. 10% of **3),** isolated by desalting on a NAP cartridge (Fig 8b).

### Detailed description of the invention

The invention is described in detail in connection with examples 1 to 8.

Example 1 illustrates the preparation of a nucleoside analogue according to the invention which analogue is immobilized and sugar- and nucleobase-protected in a way suitable for the automated synthesis of RNA-sequences. The shown analogue is a correspondingly protected and immobilized 2'-deoxy-2'-thioadenosine nucleoside.

Example 2 illustrates the preparation of an RNA-sequence analogue according to the invention and comprising the nucleoside analogue prepared according to Example 1 as a 3'-terminal nucleoside.

Example 3 illustrates the preparation of a tRNA analogue according to the invention and comprising the nucleoside prepared according to Example 1 as 3'-terminal nucleoside, the preparation comprising a template-assisted, enzyme-mediated ligation of a truncated tRNA with an RNA-sequence analogue prepared according to Example 2.

Example 4 illustrates the preparation of suitably activated amino acid derivatives applicable for aminoacylations of an RNA-sequence or nucleoside/nucleotide analogue according to the invention. The shown amino acid derivatives are thiophenylesters.

Example 5 illustrates the spontaneous aminoacylation of a 8mer RNA-sequence analogue according to the invention with an amino acid thiophenylester. The shown RNA-sequence analogue is prepared according to Example 2 and contains the 3'-terminal 2'-deoxy-2'-thioadenosine nucleoside prepared according to Example 1. The RNA-sequence analogue is aminoacylated using the amino acid derivative prepared according to Example 4.

Example 6 illustrates the aminoacylation of a 8mer RNA-sequence analogue according to the invention with an amino acid thiophenylester and the subsequent selective cleavage of the diacylated derivative to the 3'-O-monoacylated RNA-sequence analogue. The shown RNA-sequence analogue is prepared according to Example 2 and contains the 3'-terminal 2'-deoxy-2'-thioadenosine nucleoside prepared according to Example 1. The RNA analogue is aminoacylated using the amino acid derivative prepared according to Example 4.

Example 7 illustrates that RNA-sequence analogues according to the invention can be aminoacylated, hydrolyzed and re-aminoacylated several times under biologically relevant conditions, without being degraded or otherwise rendered inactive. The shown RNA-sequence analogue is a 8mer RNA-sequence prepared according to Example 2 and contains the 3'-terminal 2'-deoxy-2'-thioadenosine nucleoside prepared according to Example 1. The RNA-sequence analogue is aminoacylated using the amino acid derivative prepared according to Example 4.

Example 8 illustrates that the relevant 3'-O-aminoacylated RNA-sequence analogue can be obtained in situ, under biologically relevant conditions from the diacylated RNA-sequence analogue. The shown RNA-sequence analogue is a 8mer RNA-sequence prepared according to Example 2 and contains the 3'-terminal 2'-deoxy-2'-thioadenosine nucleoside prepared according to Example 1. The RNA analogue is aminoacylated using the amino acid derivative prepared according to Example 4.

### Example 1: Preparation of an immobilized, sugar- and nucleobase-protected 2'-deoxy-2'-thioadenosine derivative (nucleoside analogue according to the invention)

An immobilized, sugar- and nucleobase-protected 2'-deoxy-2'-thioadenosine derivative **11** was prepared from the protected adenosine derivative **6** (prepared as described in [16]) in the five step method according to the following scheme. 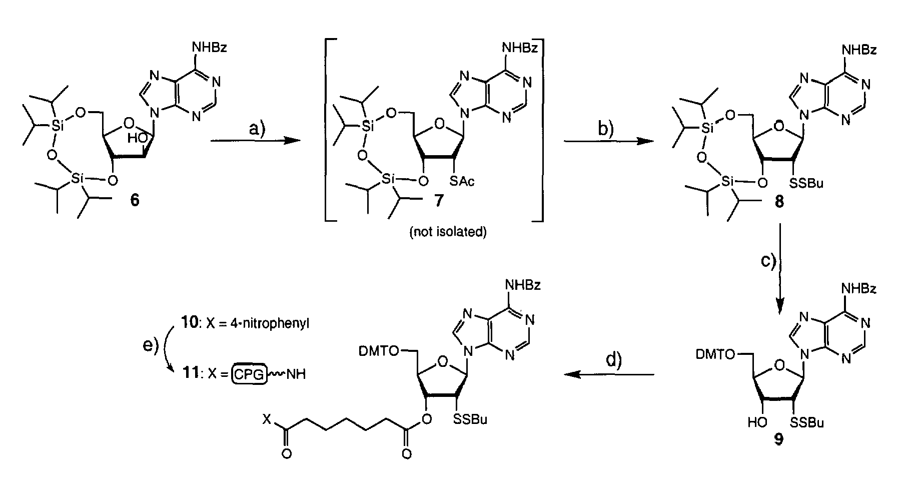 wherein, (a) is 1) (CF₃SO₂)₂O, pyridine, CH₂Cl₂, 0°→-10°, **6** was prepared according to [16] and 2) KSAc, DMSO, rt; (b) is 1) 0.2M NaOH, THF:MeOH:H₂O 5:4:1, 4° and 2) ⁿBuSH, Et₃N, SO₂Cl₂, CH₂Cl₂, 0°; (c) is 1) HF.Py (1:1), -78° to rt and 2) (MeO)₂TrCl, py, rt; (d) is Bis(4-nitrophenyl)heptanedioate, Py, DMAP, r.t; (e) is long-chain-alkylamino CPG, ⁱPr₂NEt, DMF, rt. The used abbreviations mean: Bz = benzoyl, Ac = acetyl, Bu = butyl, DMT = (4,4'-dimethoxy)trityl; CPG = long chain alkylamino controlled pore glass (solid support used for automated oligonucleotide synthesis).

For preparing the immobilized and protected 2'-deoxy-2'-thio-nucleoside **11,** a suitably protected nucleoside **6** (prepared according to [16]) is activated as a 2'-*O-*triflate derivative, which is transformed into intermediate **7** with the salt of a thiocarbonic acid. The resulting thioester **7** is hydrolized and the liberated 2'-thiol-group is reacted with an activated thiol (e.g. activated n-butanthiol or tert.-butylthiol) to give the corresponding disulfide **8.** By protecting group manipulation under standard conditions, the protected 2'-deoxy-2'-thioadenosine **9** is obtained, to which a suitable linker (e.g. an activated diester) is connected, resulting in the formation of the active ester **10,** which is finally transformed into the immobilized and protected nucleoside analogue **11.** The butyl-disulfide group constitutes a suitable protecting group for protecting the 2'-*S*-group during automated synthesis of RNA-sequences and during ligation of the nucleoside analogue or RNA fragments containing this nucleoside analogue to truncated tRNA. The butyl-thioyl-group is easily cleaved from the 3'-terminal nucleoside block of a RNA-sequence by reacting it in the way described in Example 5 and illustrated in Figs. 2a and 2b.

Other suitable protection groups for the 2'-thiol group are isoproyl-disulfide, linear-, branched or cyclic alkyl-disulfides containing 1 to 12 C-atoms and possibly containing heteroatoms, in particular oxygen, or substituted or non-substituted phenyl-disulfide groups.

For preparing 3'-deoxy-3'-thionucleosides the above described synthesis is suitably adapted.

### Example 2: Preparation of an RNA-sequence analogue containing a 3'-terminal 2'-deoxy-2'-thioadenosine nucleoside (RNA-sequence analogue according to the invention)

RNA-sequences (e.g. 2, 4, or 8mers) containing a 3'-terminal 2'-deoxy-2'-thioadenosine nucleoside being 2'-*S*-protected were prepared from the immobilized 2'-deoxy-2'-thioadenosine derivative prepared according to Example 1 and TOM-phosphoramidites under the standard conditions on a DNA-synthesizer as described in [17]. After conventional deprotection (MeNH₂ in H₂O/EtOH, followed by TBAF in THF), desalting on NAP-cartridges and purification by anion-exchange chromatography pure products were obtained in good yields (ca. 1 mg 8mer from a 1µmol-synthesis). The RP HPLC trace and the ESI-MS spectrum of a 8mer modified RNA-sequence 1, prepared according to this description, are shown in Fig. 2a and Fig. 7a, respectively.

Any other known DNA or RNA synthesis protocol (chemical or enzymatic) is applicable in the same way. RNA-sequence analogues containing a 3'-terminal 3'-deoxy-3'-thioadenosine nucleoside are prepared in an analogue manner.

### Example 3: Preparation of a tRNA analogue containing a 3'-terminal 2'-deoxy-2'-thioadenosine nucleoside (tRNA analogue according to the invention)

The tRNA analogue was prepared by using a template assisted, enzyme-mediated ligation strategy described in [18]. A truncated tRNA (68mer) transcript was incubated with equimolar amounts of an 8mer RNA-fragment containing a protected 3'-terminal 2'-deoxy-2'-thioadenosine nucleoside prepared according to Examples 1 and 2 and a complementary 14mer 2'-*O*-MeRNA-template. In the presence of ATP and T4-DNA-ligase the corresponding ligation product was formed in excellent yield and was isolated by anion exchange HPLC. The reaction is illustrated by Figs. 1a and 1b.

Any other, known ligation method (chemical or enzymatic, with or without usage of a template) may be used instead of the above shortly described method using a template. tRNA analogues containing a 3'-terminal 3'-deoxy-3'-thioadenosine nucleoside are prepared in the same way.

### Example 4: Preparation of an activated amino acid derivative suitable for aminoacylation of the nucleic acid sequence or nucleoside/nucleotide analogue according to the invention

An amino acid derivative which is activated in a way suitable for application in the 3'-*O*-aminoacylation reaction of the nucleic acid sequence or nucleoside/nucleotide analogue according to the invention is e.g. a thiophenylester, which is e.g. prepared according to the following scheme illustrating the preparation of thiophenylesters of phenylalanine (thiophenylester **5)** and Leucin (thiophenylester **16) :** 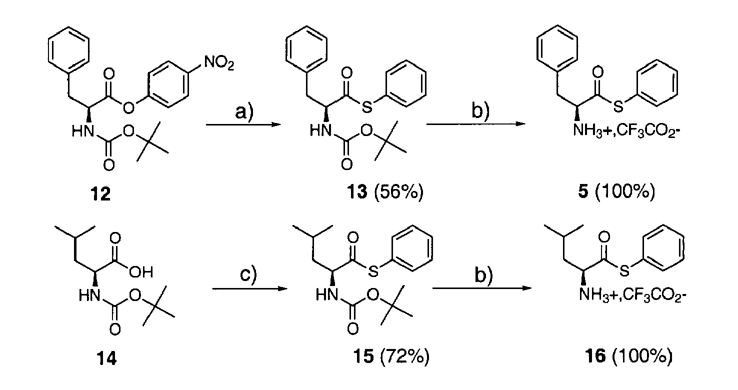 Wherein a) is thiophenol, Py, CH₂Cl₂, 25°; b) is CF₃COOH, CH₂Cl₂, 25°; and c) is 1. BOP, Et₃N, DMF, 25° and 2. Thiophenol, Py, r.t.

The above reactions can be carried out with other aminoacids also. Other suitably activated amino acid derivatives are thioalkylesters, nitrophenylester, pentafluorophenylester, N-hydroxy-succinimidester, or corresponding anhydrides (mixed or symmetrical).

### Example 5: Spontaneous aminoacylation of an RNA-sequence containing a 3'-terminal 2'-deoxy-2'-thioadenosine nucleoside with an amino acid thiophenylester

Model experiments for the spontaneous 3'-*O*-aminoacylation were carried out with a 8mer RNA-sequence 1 containing a 3'-terminal 2'-deoxy-2'-thioadenosine nucleoside prepared according to Examples 1 and 2 and an amino acid thiophenylester 5 prepared according to Example 4.

In a first step, the remaining disulfide group of 1 was cleaved with a water-soluble phosphine at different pH values (pH 3.7 - 7.4). This reaction proceeded cleanly and was complete after 30 min at room temperature. Figs. 2a and 2b illustrate this reaction, carried out at pH 7.4.

In a second step, the reaction mixture was incubated with an excess of phenylalanine phenylthioester **(5)** under a variety of different conditions (pH 3.7 - 7.4; 25° and 37°); after only 10 min almost quantitative product formation could be observed by HPLC and ESI MS. Under all reaction conditions, the *O*-monoacylated and the *S,O*-diacylated product were formed (however in different ratios, see Table 1). In Figs. 3a to 3c the course of such a reaction, carried out at pH 7.4 and 37° is documented.

The two products **3** and **4** were isolated and characterized by ESI MS (Figs. 8a and 8b).

Similar reactions occur on esterification of an RNA-sequence containing a 3'-terminal 3'-deoxy-3'-thioadenosine nucleoside with an activated amino acid derivative.

### Example 6: Preparation and isolation of an RNA-sequence containing a 3'-terminal 2'-deoxy-2'thioadenosine nucleoside esterified with an L-phenylalanine

For the preparation and isolation of monoacylated RNA-sequences, containing a 3'-terminal 2'-deoxy-2'thio group, the reaction conditions were optimized at pH 5.0, where aminoacylated RNAs are considerably stable [8]. The corresponding reaction is illustrated in Figs 4a to 4c. In order to minimize the formation of side products, the deprotection and the aminoacylation reactions were carried out simultaneously. The disulfide protected RNA-sequence **1** (Fig. 4a) was incubated with TCEP and the activated amino acid **(5)** at pH 5.0 and 37° for 30 min, after which a RP-HPLC analysis revealed clean and almost quantitative formation of the aminoacylated products **3** and **4** (Fig. 4b). The selective cleavage of the thioester bond (reaction **4→3),** was achieved by treating this reaction mixture for 30 min at 25° with NaN₃ (Fig. 4c). Finally, the monoacylated product **3** was isolated in almost pure form by chromatography on a size-exclusion cartridge, which removed all non-oligonucleotide products.

### Example 7: Course of the aminoacylation reaction of an RNA-sequence containing a 3'-terminal 2'-deoxy-2'-thioadenosine nucleoside with an excess of L-phenylalanine thiophenylester under biologically relevant conditions

In order to determine the ability of RNA-sequences, containing a 3'-terminal 2'-deoxy-2'-thio group, to be employed in catalytic amounts during *in vitro* translation reactions, the course of an aminoacylation reaction carried out at pH 7.4, 37° and with 12 equiv. of the activated amino acid **(5)** has been investigated. The corresponding reaction is illustrated by Figs. 5a and 5b.

After 2 min already, almost quantitative formation of the mono- and diacylated RNA-sequences **3** and **4** was observed (Fig. 5a). Only after about 50 min, they were slowly converted back to the starting material **2** by hydrolytic cleavage. The occurrence of a plateau between 2 and 50 min indicates the transient nature of the aminoacylated species **3** and **4,** which are constantly hydrolyzed and re-acylated, until the activated amino acid is consumed (Fig. 5b).

### Example 8: Behaviour of a mixture of RNA-sequences containing a 3'-terminal 2'-deoxy-2'-thioadenosine nucleoside esterified with one or two L-phenylalanines, respectively, under biologically relevant conditions

In order to determine the availability of the monoacylated RNA-sequence under *in vitro* translation conditions, the individual kinetic parameters of hydrolysis and the time-dependant composition of the different aminoacylated species has been determined. The corresponding reaction is illustrated by Figs. 6a to 6c.

A 3:1 mixture of the di- and monoacylated RNA-sequences **3** and **4** was prepared and isolated by desalting on a size-exclusion cartridge (Fig. 6a). This mixture was then incubated at pH 7.4 and 37°. At different time intervals, samples were withdrawn and analyzed by RP HPLC (Fig. 6b and 6c). It was found that hydrolysis of the diacylated **4** to the monoacylated compound **3** occurs faster than the complete hydrolysis of the latter to the non-acylated **2;** this results in a predominant occurrence of the biologically relevant monoacylated RNA-sequence **3** after a short incubation under these conditions (Fig. 6c).

At pH 7.4 and 37°, the hydrolysis of **3** to **2** occurs with a rate constant of k = 0.075 min⁻¹ and the hydrolysis of **4** to **3** occurs with a rate constant of k = 0.15 min⁻¹. At 25°, the rate constant for the hydrolysis of **3** was k = 0.02 min⁻¹, which is about twice the value for an adenosine nucleotide esterified with L-Phe [9].

### Literature

[1] P.E. Dawson, T.W. Muir, I. Clark-Lewis, S.B. Kent, *Science* **1994,** *266,* 776; A. Bernardi, L. Manzoni, *Chemtracts* **2000,** *13,* 588; P.E. Dawson, *Methods in Enzymology* **1997,** *287*, 34.
[2] W. Mothes, B. Jungnickel, J. Brunner, T.A. Rapoport, *J. Cell Biol*. **1998,** *142,* 355; W. Mothes, S.U. Heinrich, R. Graf, I, Nilsson, G. von Heijne, J. Brunner, *Cell*, **1997,** *89*, 523; S. High, B. Martoglio, D. Görlich, S.S.L. Andersen, A.J. Ashford, A. Giner, E. Hartmann, S. Prehn, T. A. Rapoport, B. Dobberstein, J. Brunner, *J Biol Chem*. **1993,** *268*, 26745; G. Turcatti, K. Nemetz, M. Edgerton, U. Meseth, F. Talabot, M. Peitsch, J. Knowles, H. Vogel and A. Chollet *J. Biol. Chem*. **1996,** *271,* 19991.
[3] H.H. Chung, D.R. Benson, V.W. Cornish, P.G. Schultz *Proc. Natl. Acad. Sci. USA* **1993,** *90*, 10145; D. Mendel, J.A. Ellman, Z. Chang, D.L. Veenstra, P.A. Kollman, P.G. Schultz, *Science* **1992,** *256*, 1798.
[4] D. Mendel, J.A. Ellmann, P.G. Schultz, *J Am. Chem. Soc.* **1991,** *113*, 2758.
[5] B. Robson, J. Garnier, *Introduction to Proteins and Protein Engineering,* **1988,** Elsevier, New York.
[6] S.M. Hecht, *Ace. Chem. Res.* **1992,** *25,* 545 (and references cited therein).
[7] C.J. Noren, S.J. Anthony-Cahill, M.C. Griffith, P.G. Schultz *Science* **1989,** *244*, 182; S.A. Robertson, J.A. Ellman, P.g. Schultz *J. Am. Chem. Soc.* **1991,** *113*, 2722.; T.G. Heckler, L. Chang, Y. Zama, T. Naka, M.S. Chorghade, S.M. Hecht *Biochemistry* **1984,** *23*, 1468; G. Baldini, B. Martoglio, A. Schachenmann, C. Zugliani, J. Brunner, *Biochemistry* **1988,** *27*, 7951.
[8] M. van Cleve in S.M. Hecht, *Bioorganic Chemistry (Nucleic Acids),* **1996,** Oxford University Press, New York.
[9] P. Schimmel, D. Soll, J.A. Abelson, *Transfer RNA: Structure, Properties, and Recognition,* **1979,** Cold Spring Harbor Press, Cold Spring Harbor.
[10] D.R. Liu, T.J. Magliery, M. Pastrnak, P.G. Schultz, *Proc. Natl. Acad. Sci. USA* **1997,** *94*, 10092; L. Wang, A. Brock, B. Herbrich, P.G. Schultz, *Science* **2001,** *292*, 498; A.K. Kowal, C. Kohrer, U.L. RajBhandary, *Proc. Natl. Sci. **2001,** 98**,** 2268**.***
[11] N. Lee, Y. Bessho, K. Wei, J.W. Szostak, H. Suga, *Nature Struct. Biol*. **2000,** *7,* 28; K. Ramaswamy, K. Wei, H. Suga, *Nucl. Acids Res.* **2002,** *30,* 2162; Y. Bessho, D.R.W. Hodgson, H. Suga, *Nature Biotech.* **2002,** *20,* 723.
[12] J.D. Bain, C. Switzer, A.R. Chamberlin, S.A. Benner, *Nature* **1992,** *356,* 537.
[13] I. Hirao, T. Ohtsuki, T. Fujiwara, T. Mitsui, T. Yokogawa, T. Okuni, H. Nakayama, K. Takio, T. Yabuki, T. Kigawa, K. Kodama, T. Yokogawa, K. Nishikawa, S. Yokoyama, *Nature Biotechnology* 2002, 177.
[14] M. Sisido, T. Hohsaka, *Bull. Chem. Soc. Jpn.* 1999, 72, 1409; M. Sisido, T. Hohsaka, *Appl. Microbiol. Biotechnol.* **2001,** *57,* 274; T. Hohsaka, Y. Ashizuka, H. Murakami, M. Sisido, *Nucleic Acids Research,* 2001, 29, 3646.
[15] T. Hohsaka, Y. Ashizuka, H. Sasaki, H. Murakami, M. Sisido, *J. Am. Chem. Soc.* **1999,** *121*, 12194.
[16] J.H.Marriott, M. Mottahedeh, C.B. Reese, *Carbohydrate Res.* **1991***, 216, 257.*
[17] S. Pitsch, P.A. Weiss, X. Wu, D. Ackermann, T. Honegger, *Helv. Chim. Acta* **1999,** *82,* 1753; S. Pitsch, P.A. Weiss, L. Jenny, X. Wu, A. Stutz, *Helv. Chim. Acta* **2001,** *84*, 3773.
[18] A. Stutz, "Methoden zur Ligation von Oligoribonukleotiden und ihre Anwendung bei der Totalsynthese aminoacylierter tRNAs", Diss. ETH Zürich, 2003.
[19] A. Stutz, S. Pitsch, C. Höbartner, *Helv. Chim. Acta* **2000,** *83,* 2477.
[20] K. Ninomiya, T. Minohata, M. Nishimura, M. Sisido, *J. Am. Chem. Soc* **2004***, 126*, 15984.

## Claims

1. Nucleoside/nucleotide analogue, which is a 2'-deoxy-2'-thionucleoside or - nucleotide or a 3'-deoxy-3'-thio-nucleoside or -nucleotide.

2. Nucleic acid sequence analogue comprising a 3'-terminal 2'-deoxy-2'-thionucleoside or 3'-deoxy 3'-thionucleoside.

3. Nucleic acid sequence analogue according to claim 2, which is a tRNA analogue.

4. Nucleic acid sequence or nucleoside/nucleotide analogue according to one of claims 1 to 3, wherein the 2'-deoxy-2'-thionucleoside/nucleotide or the 3'-deoxy-3'-thionucleoside/nucleotide is a thioadenosine.

5. Nucleic acid sequence or nucleoside/nucleotide analogue according to one of claims 1 to 4, wherein the 3'- or 2'-thiogroup is protected by a substituted disulfide-group.

6. Nucleic acid sequence or nucleoside/nucleotide analogue according to claim 5, wherein the disulfide group is an n-butyl-disulfide group, a tert.-butyl-disulfide group, an isopropyl-disulfide group, a substituted or non-substituted phenyl-disulfide group or an alkyl-disulfide group, wherein the alkyl comprises 1 to 12 C-atoms.

7. Nucleoside/nucleotide according to one of claims 5 to 6 which is immobilized by being linked via its 3'-*O*-position or its 2'-*O*-position and via a linker to a solid phase, whose 5'-*O*-position is protected by a dimethoxytrityl-group and whose base is protected by being acylated.

8. Nucleic acid sequence analogue according to one of claims 1 to 4, wherein the 3'-*O*- or 2'-*O*-position is aminoacylated.

9. Nucleic acid sequence analogue according to one of claims 1 to 4, wherein the 3'-*O*- and the 2'-*S*-position or the 2'-*O*- and the 3'-*S*-position are both aminoacylated.

10. Nucleic acid sequence analogue according to one of claims 8 or 9, wherein the aminoacylated thionucleoside is stabilized by an elongation factor.

11. Method for preparing a 3'-*O*-aminoacylated or a 3'-*O*,2'-*S*-diaminoacylated derivative of a 2'-deoxy-2'-thionucleoside/nucleotide or of a 3'-*S*,2'-*O-*diaminoacylated derivative of a 3'-deoxy-3'-thionucleoside/nucleotide or of a nucleic acid sequence analogue comprising one of said thionucleosides as a 3'-terminal nucleoside, the method comprising the step of reacting the 2'-deoxy-2'-thionucleoside or the 3'-deoxy-3'-thionucleoside or the nucleic acid sequence analogue with an excess of a suitably activated amino acid derivative.

12. Method according to claim 11, wherein the activated amino acid derivative is a thioester, an N-hydroxy-succinimidester or an anhydride.

13. Method according to claim 12, wherein the thioester is a thioalkylester, a thiophenylester, a nitrophenylester or a pentafluorophenylester.

14. Method according to one of claims 11 to 13, wherein the nucleic acid sequence analogue is a tRNA analogue and wherein the method further comprises the step of stabilizing the analogue with an elongation factor.

15. Method according to one of claims 11 to 14 wherein the step of aminoacylating or di-aminoacylating is carried out simultaneously with a step of deprotecting the 2'-thiol or 3'-thiol group.

16. Method for preparing a 2'-deoxy-2'-thionucleoside/nucleotide or a 3'-deoxy-3'-thionucleoside/nucleotide, the method comprising the steps of preparing from a sugar- and amino-protected nucleoside or nucleotide derivative a 2'- or 3'-thioester-derivative and hydrolizing the thioester.

17. Method according to claim 16 and further comprising the step of protecting the 2'- or 3'-thiogroup by reacting it with a thiol to form a disulfide.

18. Method for preparing a tRNA analogue comprising a 3'-terminal 2'-deoxy-2'-thioadenosine or 3'-deoxy-3'-thioadenosine, the method comprising the steps of ligating a protected 2'-deoxy-2'-thioadenosine or a 3'-deoxy-3'-thioadenosine as a 3'-terminal nucleoside to an RNA-sequence and ligating the resulting RNA-sequence analogue to a correspondingly truncated tRNA.

19. Use of a tRNA analogue according to one of claims 1 to 4 or 8 to 10 in ribosome-mediated, site-specific incorporation of unnatural amino acids into proteins.

20. Use according to claim 19, wherein the incorporation is carried out *in vitro* or in cultured cells or micro-organisms.
